# FASCICULE DE BREVET EUROPEEN

(11) **EP 3 911 347 B1**
(45) Date de publication et mention de la délivrance du brevet: **04.10.2023**
(21) Numéro de dépôt: 20700172.8
(22) Date de dépôt: 13.01.2020
(51) Int. Cl.: A61K 36/899, A61K 36/15, A61K 36/42, A61K 31/355, A61P 13/02, A61P 13/10, A61P 13/00

(54) **COMPOSITION POUR SON UTILISATION DANS LE TRAITEMENT DE L'INCONTINENCE URINAIRE CHEZ LA FEMME**
ZUSAMMENSETZUNG ZUR VERWENDUNG BEI DER BEHANDLUNG VON HARNINKONTINENZ BEI FRAUEN
COMPOSITION FOR FOR USE IN THE TREATMENT OF URINARY INCONTINENCE IN WOMEN

(30) Priorité: 18.01.2019 FR 1900478
(43) Date de publication de la demande: 24.11.2021
(73) Titulaire: AXEEN PHARMA S.A.R.L., 98000 Monaco (MC)
(72) Inventeur: MORRA, Sossio, 98000 Monaco (MC)
(74) Mandataire: Axe PI
(86) Numéro de dépôt international: PCT/EP2020/050671
(87) Numéro de publication internationale: WO 2020/148224

(56) Documents cités:
- FR-A1- 3 055 215
- FR-A1- 3 068 605
- Anonymous: "History of Changes for Study: NCT03438422", , 22 octobre 2018 (2018-10-22), XP055620551, Extrait de l'Internet: URL:https://clinicaltrials.gov/ct2/history /NCT03438422?V_2=View#StudyPageTop [extrait le 2019-09-10]
- William Faloon: "A Breakthrough in the Relief of Overactive Bladder and Urinary Incontinence UNDERLYING CAUSE OF URINARY MISERIES", , 1 novembre 2008 (2008-11-01), XP055620527, Extrait de l'Internet: URL:https://pdfs.semanticscholar.org/f44f/ 9a1cb2cd8a6d1d559d7c29ed79cc22579398.pdf [extrait le 2019-09-10]

## Description

### Domaine technique

La présente invention concerne une composition à base d'extraits de plantes pour son utilisation dans le traitement et/ou la prévention des symptômes du bas appareil urinaire (SBAU).

Les symptômes du bas appareil urinaire (SBAU ou LUTS en anglais pour « Lower Urinary Tract Symptoms ») sont fréquents chez les hommes et les femmes de tous âges. La prévalence et la gravité des SBAU augmentent avec l'âge et constituent un problème majeur au sein de la population vieillissante. Il est désormais admis que les SBAU constituent un groupe de symptômes progressifs, liés à l'âge, non spécifiques du sexe ou d'un organe, et qui comportent une combinaison de symptômes des phases de remplissage, mictionnelle et post mictionnelle.

L'incontinence urinaire en est un des symptômes principaux. Il est connu (DeMaagd et al., « Management of Urinary Incontinence », P&T®, juin 2012, Vol. 37 No. 6) qu'environ 10 millions de patients aux États-Unis ont une incontinence urinaire. Par exemple, en 2007, on estime que plus de 25 millions de personnes aux États-Unis ont connu des épisodes d'incontinence urinaire. Il apparaît que la prévalence de l'incontinence urinaire est plus élevée chez les femmes que chez les hommes de 80 ans ou moins, mais les hommes et les femmes sont touchés presque tout aussi après les 80 ans.

Les SBAU peuvent avoir des causes multiples situées au niveau du tractus urinaire ou à distance. Les SBAU peuvent résulter d'infections et d'inflammations (par exemple cystite, prostatite), d'une affection d'origine structurale (par exemple calculs, tumeurs, prolapsus, hypertrophie bénigne de la prostate), d'un problème médical (par exemple diabète sucré, sclérose en plaques) ou encore de consommation de drogues (par exemple anticholinergiques, opioïdes).

Bien qu'en général les SBAU ne causent pas de maladies graves, ils constituent un motif fréquent de consultation médicale, peuvent affecter durement la qualité de vie et être une indication d'une affection grave de l'appareil urinaire. Ces SBAU peuvent être responsables de complications et être par exemple associés à une dysfonction sexuelle, une dépression, une hypertension et une anxiété.

De par la complexité étiologique, les traitements des SBAU doivent ainsi être adaptés à chaque patient, et prendre en compte le type et l'intensité des symptômes, leur progressivité, l'existence de comorbidités, et la qualité de vie des patients. En outre, l'opinion personnelle des patients doit être prise en considération.

Toutes les personnes qui ont des SBAU ne nécessitent pas obligatoirement de traitement.

Il peut néanmoins être nécessaire de recourir à des traitements médicamenteux voire chirurgicaux pour traiter les SBAU.

### Technique antérieure

Le traitement médicamenteux de choix pour un patient ayant des symptômes modérés à sévères, responsables d'un retentissement sur la qualité de vie, est un alpha-bloquant en monothérapie. Ce sont des antagonistes des récepteurs alpha adrénergiques. Ils agissent par relaxation des fibres musculaires lisses du col vésical et de la prostate chez l'homme et/ou et de l'urètre proximal chez la femme. Leur action est rapide et stable sur plusieurs années, avec une amélioration significative des SBAU.

Néanmoins, les alpha-bloquants présentent des effets secondaires importants tels qu'une hypotension orthostatique, des céphalées, des vertiges ou encore des troubles de l'accommodation. Ils doivent également être prescrits avec précaution chez les patients âgés, coronariens, et en cas de traitement antihypertenseur associé. Ils sont contre-indiqués avant chirurgie de la cataracte ou en cas d'antécédent d'hypotension orthostatique.

Les inhibiteurs de la 5-alpha réductase (I5AR) sont des médicaments de deuxième intention. Ils sont utilisés plus particulièrement chez l'homme et bloquent la conversion de la testostérone en dihydrotestostérone (DHT), qui est le métabolite actif sur la croissance prostatique. Leur efficacité a été démontrée sur les symptômes, le débit, la diminution du risque de rétention aiguë d'urines, la diminution de la nécessité de recours à la chirurgie, et la diminution de 20% du volume prostatique.

Toutefois, leur action est lente, entre 3 et 6 mois et engendrent des troubles sexuels (diminution de la libido, troubles de l'érection, gynécomastie).

L'association thérapeutique entre un alpha-bloquant et un inhibiteur de la 5-alpha-réductase est plus efficace à long terme sur la symptomatologie urinaire que chacune des monothérapies, mais les effets secondaires se cumulent.

On connaît d'autres traitements utilisant des anti-cholinergiques (anti-muscariniques) ou encore des inhibiteurs de phosphodiestérase de type 5 (IPDE5).

Toutefois, ils ne présentent qu'une efficacité limitée, par exemple aux urgenturies pour les anti-cholinergiques tout en entraînant des effets indésirables tels que la sécheresse buccale, la constipation, la confusion, et surtout la majoration de la dysurie.

Le collagène est également connu pour être utilisé notamment dans le traitement de l'incontinence urinaire chez la femme.

Néanmoins, le collagène est administré par injection ce qui nécessite une intervention et peut réduire l'observance.

La phytothérapie est également utilisée dans le traitement des SBAU. Ce sont des traitements à base d'extraits de plantes qui présentent une excellente tolérance du fait de l'absence d'effets secondaires. Les molécules les plus utilisées sont extraites de *Pygeum africanum* et de *Serenoa repens.*

Néanmoins, même si la littérature est contradictoire à leur sujet, leur efficacité sur les SBAU bien que significative, reste modeste. Leur association aux autres traitements n'est pas préconisée.

A cet effet, on connaît des compositions pour maintenir la santé de la prostate chez l'homme.

On connaît par exemple les publications de H.G. Preuss et al. ("A Critical Review of Cernitin™ for Symptomatic Relief Of Lower Urinary Tract Symptoms (LUTS) in Men", RESEARCH COMMUNICATIONS IN PHARMACOLOGY AND TOXICOLOGY, 2013) ou W. G. Chambliss et al. ("Flower Pollen Extract and its Effects on Urinary Support, Bladder and Smooth Muscle Contents", 2013) qui divulguent l'activité de CERNITIN^{™} (= GRAMINEX^{®} Flower Pollen Extract) chez des hommes ayant une hypertrophie bénigne de la prostate pouvant occasionner des symptômes urinaires du bas appareil ou présentant d'autres désordres liés à la prostate. CERNITIN^{™} est un mélange de pollen de seigle (*Secale céréale L.*), de fléole des près (*Phleum pratense*) et de maïs (*Zea mays L*.), comprenant un extrait aqueux et un extrait huileux de pollen dans un ratio 20:1.

D'autres compositions à base de GRAMINEX^{®} comprenant par exemple notamment un extrait de graines de citrouille contenant 25% d'acides gras (« Prostate Support - Weil Vitamin Advisor», 2016) ou un extrait de graines de citrouille standardisé à 10% d'acides gras et de la vitamine E (PROSTACERN^{™}) sont connues pour supporter les problèmes liés à la prostate chez les hommes.

De tels produits sont donc essentiellement destinés aux hommes présentant des problèmes liés à la prostate.

A cet effet, les hommes peuvent rencontrer des problèmes d'incontinence mais les raisons diffèrent de celles des femmes. Il s'agit principalement :
- des troubles liés à la prostate telle que l'augmentation de son volume (hypertrophie prostatique) qui compresse l'urètre. Le sujet masculin souffre alors de la survenue de gouttes retardataires voire de fuites d'urine nocturnes ;
- de la prostactétomie : qui est l'ablation de la prostate ;
- de problèmes de santé comme la tumeur de l'urètre ou la sténose urétrale qui se définit par le rétrécissement de l'urètre et qui obstrue l'écoulement urinaire habituel.

Toutefois, même si les désordres liés à la prostate de manière générale peuvent résulter en des symptômes du bas appareil urinaire, de telles compositions n'apparaissent pas, prima facie, être directement indiquées pour le traitement et/ou la prévention des SBAU, et plus particulièrement de l'incontinence urinaire, chez la femme.

On connaît néanmoins le produit PURE^{™} Control C/P (« incontinence, frequent urination, bladder health - Women Living Naturally », 17 juin 2017) qui est dirigé vers un traitement de l'incontinence urinaire chez la femme. Il consiste en un extrait de pollen CERNITIN^{™} (mélange de pollen de seigle (*Secale céréale L.)*, de fléole des près (*Phleum pratense*) et de maïs (*Zea mays L*.) comprenant un extrait aqueux et un extrait huileux de pollen dans un ratio 20:1) et un extrait de graines de citrouille standardisé pour ne pas contenir d'acides gras mais des lignanes et des dérivés phénoliques. Il apparaît qu'un tel extrait de graines de citrouille dépourvu d'acides gras (habituellement connu pour avoir une forte teneur en acides gras) aide à maintenir une miction saine pendant la journée et la nuit après 6 semaines de traitement chez des femmes post-ménopausées.

FR 3 068 605 décrit une composition comprenant un extrait aqueux de pollen obtenu à partir d'un mélange de plantes appartenant à la famille des Poacées et des Pinacées (la composition comprenant un extrait huileux et un extrait aqueux de pollen), un extrait aqueux de graines de Cucurbita, et de la vitamine E, pour son utilisation dans le traitement des symptômes du bas appareil urinaire (SBAU), en particulier l'incontinence urinaire.

### Problème technique

Considérant ce qui précède, afin de résoudre les problèmes connus liés aux effets indésirables des traitements utilisés, et d'améliorer l'efficacité contre les SBAU, en particulier l'incontinence urinaire, et ce spécifiquement chez la femme au sens large, par exemple des femmes de 16 à 75 ans, tout en présentant une excellente tolérance, le Demandeur a développé une nouvelle composition à base d'extraits de plantes.

En outre, la maîtrise des paramètres de fabrication des extraits de plantes permet d'assurer une traçabilité, une standardisation et une reproductibilité encadrée par une spécification des extraits.

### Solution technique

La solution au problème posé par la présente invention a pour objet une composition comprenant un extrait de pollen consistant en un extrait aqueux de pollen obtenu à partir d'un mélange de : - 45% à 90% d'extrait aqueux de pollen de *Secale céréale L.* en poids du poids total de l'extrait ; - 1% à 35% d'extrait aqueux de pollen de *Zea mays L.* en poids du poids total de l'extrait ; - 0,01% à 5% d'extrait aqueux de pollen de *Pinus sylvestris L.* en poids du poids total de l'extrait ; et - 3% à 30% d'extrait aqueux de pollen de *Dactylis glomerata L.* en poids du poids total de l'extrait, un extrait aqueux de pistil de Zea *mays L.,* un extrait aqueux de graines de plante(s) appartenant au genre *Cucurbita* contenant des acides gras à une teneur de moins de 10% en poids du poids total de l'extrait, et de la vitamine E ou ses esters, pour son utilisation dans le traitement de l'incontinence urinaire chez la femme.

### Avantages apportés

Le Demandeur a pu mettre en évidence que l'utilisation de la composition objet de l'invention permet notamment la prévention ou l'amélioration des symptômes du bas appareil urinaire (SBAU) associés aux phases de remplissage, de miction et/ou de post-miction chez la femme, quel que soit son âge, et plus préférentiellement encore de l'incontinence urinaire, en particulier le traitement et/ou la prévention de l'incontinence urinaire à l'effort (IUE), de l'incontinence urinaire par urgenturie (ou impériosité) et de l'incontinence urinaire mixte (IUM), dont notamment la réduction de la fréquence mictionnelle nocturne induisant un réveil.

### Brève description des dessins

L'invention et les avantages qui en découlent seront mieux compris à la lecture de la description et des modes de réalisation non limitatifs qui suivent, illustrés au regard des dessins annexés dans lesquels :

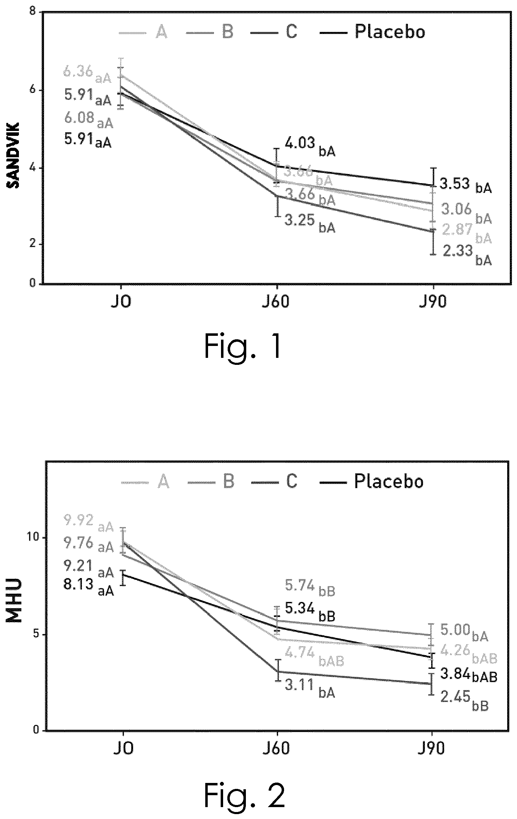

La Figure 1 représente les résultats obtenus avec l'échelle de SANDVIK de manière comparative avec différentes compositions dans le cadre de l'étude selon l'exemple 5 sur l'incontinence urinaire chez la femme avant traitement (J0), après 60 jours de traitement (J60) et après 90 jours de traitement (J90).
La Figure 2 représente les résultats obtenus avec l'échelle MHU (Mesure du Handicap Urinaire) de manière comparative avec différentes compositions dans le cadre de l'étude selon l'exemple 5 sur l'incontinence urinaire chez la femme avant traitement (J0), après 60 jours de traitement (J60) et après 90 jours de traitement (J90).

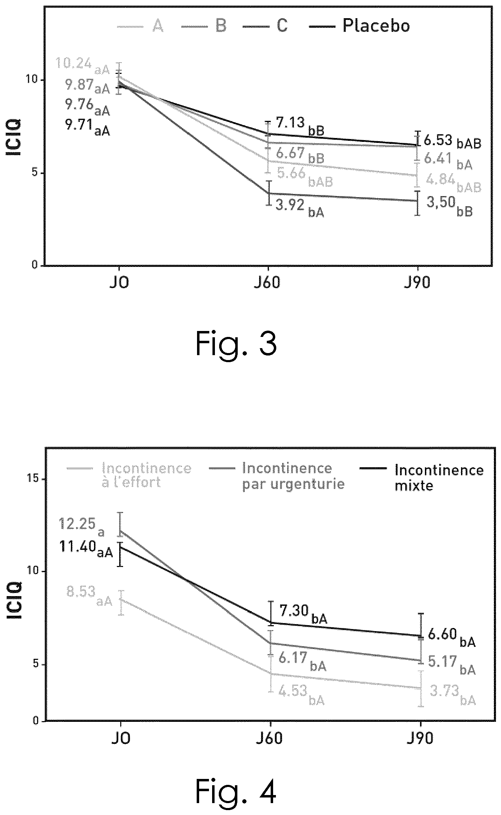

La Figure 3 représente les résultats obtenus avec l'échelle ICIQ (International Consultation on Incontinence Questionnaire) de manière comparative avec différentes compositions dans le cadre de l'étude selon l'exemple 5 sur l'incontinence urinaire chez la femme avant traitement (J0), après 60 jours de traitement (J60) et après 90 jours de traitement (J90).
La Figure 4 représente les résultats obtenus par type d'incontinence avec l'échelle ICIQ avec la composition A dans le cadre de l'étude selon l'exemple 5 sur l'incontinence urinaire chez la femme avant traitement (J0), après 60 jours de traitement (J60) et après 90 jours de traitement (J90).

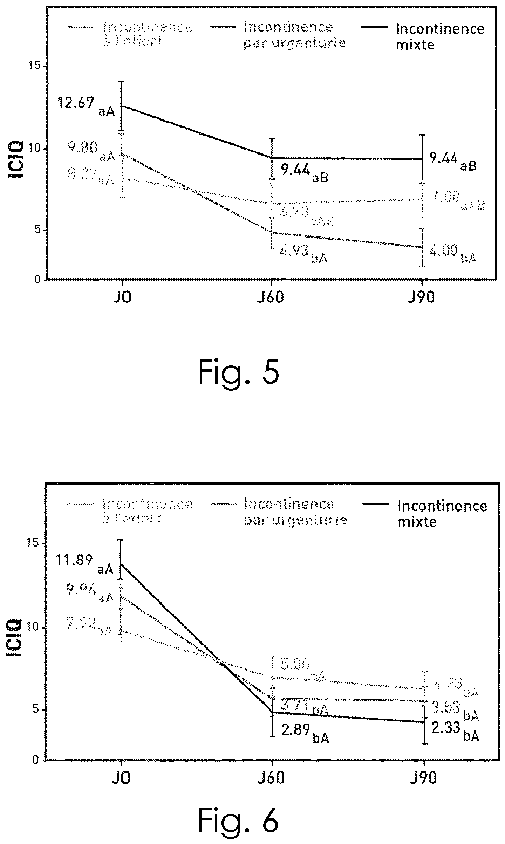

La Figure 5 représente les résultats obtenus par type d'incontinence avec l'échelle ICIQ avec la composition B dans le cadre de l'étude selon l'exemple 5 sur l'incontinence urinaire chez la femme avant traitement (J0), après 60 jours de traitement (J60) et après 90 jours de traitement (J90).
La Figure 6 représente les résultats obtenus par type d'incontinence avec l'échelle ICIQ avec la composition C dans le cadre de l'étude selon l'exemple 5 sur l'incontinence urinaire chez la femme avant traitement (J0), après 60 jours de traitement (J60) et après 90 jours de traitement (J90).

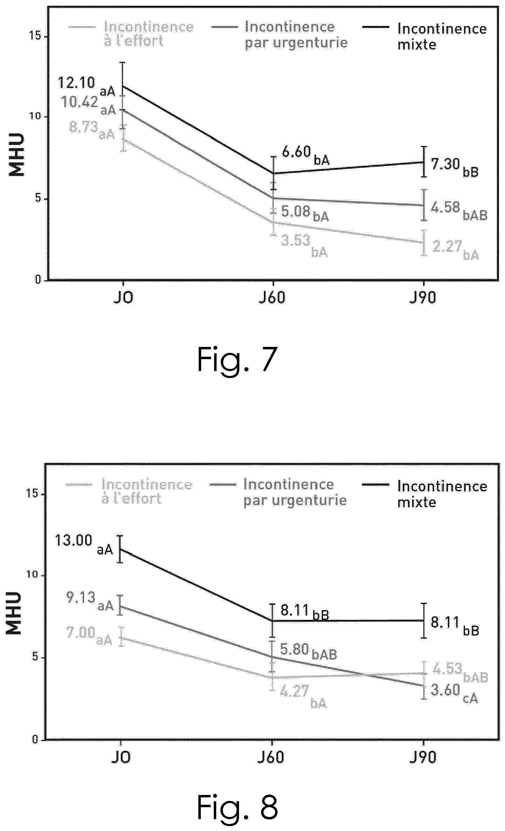

La Figure 7 représente les résultats obtenus par type d'incontinence avec l'échelle MHU avec la composition A dans le cadre de l'étude selon l'exemple 5 sur l'incontinence urinaire chez la femme avant traitement (J0), après 60 jours de traitement (J60) et après 90 jours de traitement (J90).
La Figure 8 représente les résultats obtenus par type d'incontinence avec l'échelle MHU avec la composition B dans le cadre de l'étude selon l'exemple 5 sur l'incontinence urinaire chez la femme avant traitement (J0), après 60 jours de traitement (J60) et après 90 jours de traitement (J90).

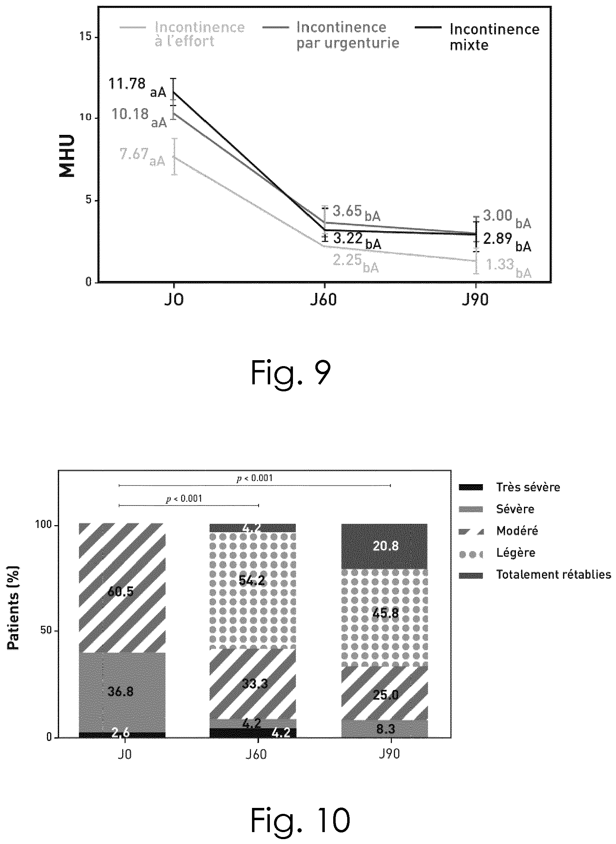

La Figure 9 représente les résultats obtenus par type d'incontinence avec l'échelle MHU avec la composition C dans le cadre de l'étude selon l'exemple 5 sur l'incontinence urinaire chez la femme avant traitement (J0), après 60 jours de traitement (J60) et après 90 jours de traitement (J90).
La Figure 10 représente les résultats obtenus avec l'échelle SANDVIK avec la composition C dans le cadre de l'étude selon l'exemple 5 sur tout type d'incontinence urinaire confondu chez la femme avant traitement (J0), après 60 jours de traitement (J60) et après 90 jours de traitement (J90).

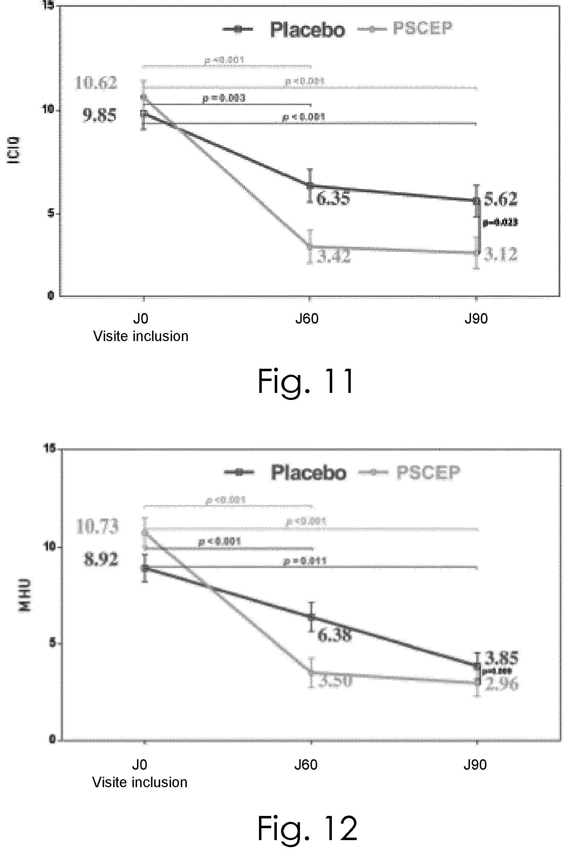

La Figure 11 représente les résultats obtenus avec l'échelle ICIQ dans le cadre de l'exemple 6 sur l'efficacité d'une composition selon l'invention (selon l'exemple 1 = PSCEP ; versus placebo) chez des femmes présentant une incontinence urinaire par urgenturie (ou impériosité) ou une incontinence urinaire mixte avant traitement (J0), après 60 jours de traitement (J60) et après 90 jours de traitement (J90).
La Figure 12 représente les résultats obtenus avec l'échelle MHU dans le cadre de l'exemple 6 sur l'efficacité d'une composition selon l'invention (selon l'exemple 1 = PSCEP ; versus placebo) chez des femmes présentant une incontinence urinaire par urgenturie (ou impériosité) ou une incontinence urinaire mixte avant traitement (J0), après 60 jours de traitement (J60) et après 90 jours de traitement (J90).

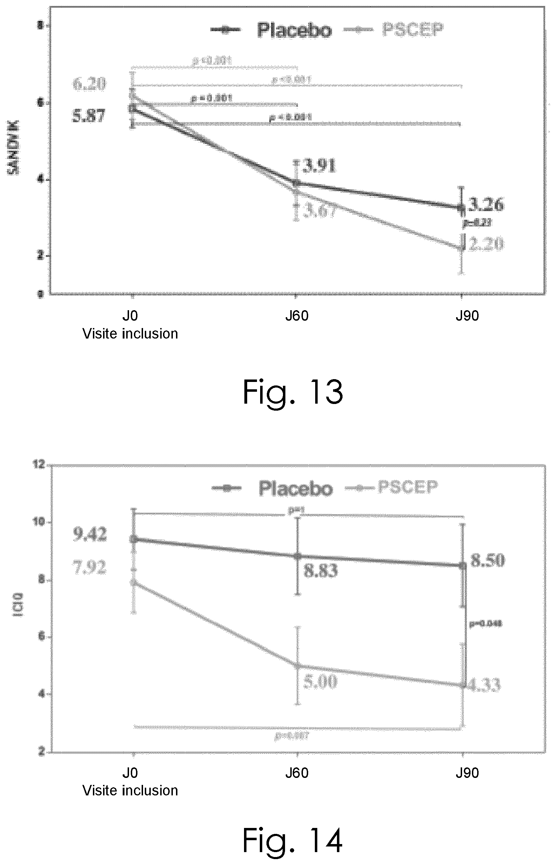

La Figure 13 représente les résultats obtenus avec l'échelle SANDVIK dans le cadre de l'exemple 6 sur l'efficacité d'une composition selon l'invention (selon l'exemple 1 = PSCEP ; versus placebo) chez des femmes présentant une incontinence urinaire par urgenturie (ou impériosité) ou une incontinence urinaire mixte avant traitement (J0), après 60 jours de traitement (J60) et après 90 jours de traitement (J90).
La Figure 14 représente les résultats obtenus avec l'échelle ICIQ dans le cadre de l'exemple 7 sur l'efficacité d'une composition selon l'invention (selon l'exemple 1 = PSCEP ; versus placebo) chez des femmes présentant une incontinence urinaire à l'effort avant traitement (J0), après 60 jours de traitement (J60) et après 90 jours de traitement (J90).

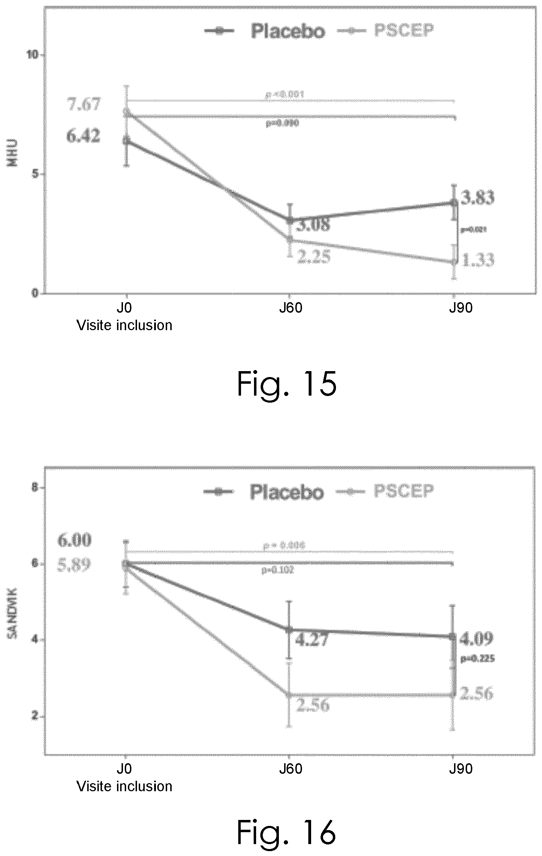

La Figure 15 représente les résultats obtenus avec l'échelle MHU dans le cadre de l'exemple 7 sur l'efficacité d'une composition selon l'invention (selon l'exemple 1 = PSCEP ; versus placebo) chez des femmes présentant une incontinence urinaire à l'effort avant traitement (J0), après 60 jours de traitement (J60) et après 90 jours de traitement (J90).
La Figure 16 représente les résultats obtenus avec l'échelle SANDVIK dans le cadre de l'exemple 7 sur l'efficacité d'une composition selon l'invention (selon l'exemple 1 = PSCEP ; versus placebo) chez des femmes présentant une incontinence urinaire à l'effort avant traitement (J0), après 60 jours de traitement (J60) et après 90 jours de traitement (J90).

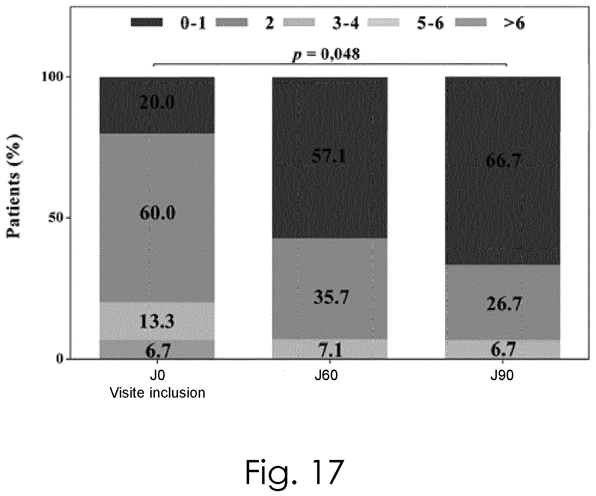

La Figure 17 représente les résultats obtenus dans le cadre de l'exemple 8 sur l'efficacité d'une composition selon l'invention (selon l'exemple 1) sur la fréquence mictionnelle nocturne induisant un réveil chez des femmes présentant une incontinence urinaire par urgenturie (ou impériosité) ou une incontinence urinaire mixte avant traitement (J0), après 60 jours de traitement (J60) et après 90 jours de traitement (J90).

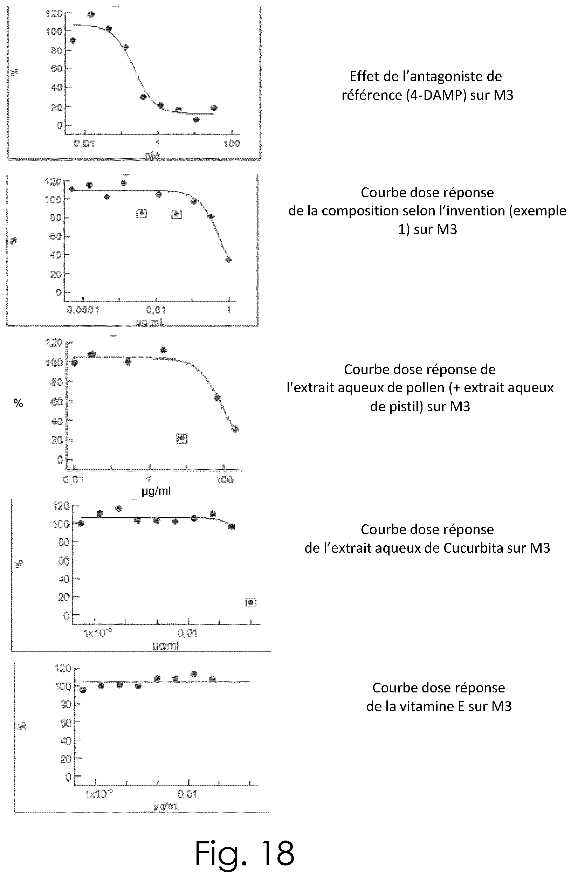

La Figure 18 représente les résultats obtenus dans le cadre de l'exemple 9 sur l'activité de la composition selon l'invention (selon l'exemple 1), de manière comparative avec chaque ingrédient actif de la composition pris indépendamment, sur le récepteur muscarinique M3.

### Description des modes de réalisation

Dans cette description, à moins qu'il ne soit spécifié autrement, il est entendu que, lorsqu'un intervalle est donné, il inclut les bornes supérieure et inférieure dudit intervalle.

L'invention est définie dans les revendications et concerne une composition utilisée dans le traitement de l'incontinence urinaire chez la femme comprenant un extrait de pollen consistant en un extrait aqueux de pollen obtenu à partir d'un mélange de : - 45% à 90% d'extrait aqueux de pollen de *Secale céréale L.* en poids du poids total de l'extrait ; - 1% à 35% d'extrait aqueux de pollen de *Zea mays L.* en poids du poids total de l'extrait ; - 0,01% à 5% d'extrait aqueux de pollen de *Pinus sylvestris L.* en poids du poids total de l'extrait ; et - 3% à 30% d'extrait aqueux de pollen de *Dactylis glomerata L.* en poids du poids total de l'extrait, un extrait aqueux de pistil de Zea *mays L.,* un extrait aqueux de graines de plante(s) appartenant au genre *Cucurbita* contenant des acides gras à une teneur de moins de 10% en poids du poids total de l'extrait, et de la vitamine E ou ses esters.

Le terme « traitement » désigne une amélioration, une prophylaxie ou un renversement d'une maladie ou d'un trouble, ou d'au moins un symptôme discernable de celui-ci. Il s'agit également d'une amélioration, d'une prophylaxie ou d'un renversement d'au moins un paramètre physique mesurable lié à la maladie ou au trouble traité, ce qui n'est pas nécessairement perceptible par le sujet. Dans un autre mode de réalisation, le terme « traitement » désigne l'inhibition ou le ralentissement de la progression d'une maladie ou d'un trouble, soit physiquement, par exemple, la stabilisation d'un symptôme discernable, physiologiquement, par exemple, la stabilisation d'un paramètre physique, ou les deux. Le terme « traitement » désigne également le retard de l'apparition d'une maladie ou d'un trouble. Dans certains modes de réalisation particuliers de l'invention, la composition d'intérêt est administrée en tant que mesure préventive. Dans ce contexte, le terme « prévention » désigne une réduction du risque d'acquisition d'une maladie ou d'un trouble spécifié.

Par extrait de pollen selon l'invention, on entend un extrait de pollen consistant en un extrait aqueux de pollen obtenu à partir d'un mélange de plantes appartenant à la famille des Pinacées et des Poacées.

Les *Poaceae,* appelée également *Gramineae,* sont une famille de plantes monocotylédones de l'ordre des Poales. Le nom d'usage est « Poacées » ou « Graminées». Cette famille, composée d'environ 12 000 espèces regroupées en 780 genres, comprend la plupart des espèces appelées communément « herbes » et « céréales ». Ce sont généralement des plantes herbacées, plus rarement ligneuses (bambous).

Comme tous les pollens anémophiles, le pollen des Poacées est de forme sphérique ou légèrement ellipsoïdale à ornementations réduites. L'aperture unique (ou pore) est ronde : c'est un des critères des Monocotylédones. Le pollen des Poacées est de petite taille et léger. La taille est de l'ordre de 40 microns. Pour les céréales, la taille est de 60 à 100 microns.

La famille des Pinacées (*Pinaceae*), ou Abiétacées, regroupe des plantes gymnospermes ; elle compte 220-250 espèces réparties en 11 genres. Ce sont des arbres ou des arbustes, des régions tempérées, soit à feuilles persistantes en aiguille ou en écailles, soit caduques comme celles des mélèzes. Dans cette famille, les espèces indigènes en France se trouvent parmi les genres *Abies* (les sapins), *Picea* (les épicéas), *Larix* (le mélèze d'Europe), *Pinus* (les pins).

Les Pinacées produisent de gros grains de pollen de manière abondante dont la taille est généralement comprise entre 40 et 100 microns. Ils sont dépourvus de pores. Les grains de pollen des pins, sapins, épicéa et cèdre possèdent deux ballonnets qui facilitent leur suspension dans l'air. Les grains de pollen des mélèzes et des douglas sont plus ou moins sphériques et sans ballonnet.

L'extrait de pollen selon l'invention consiste en un extrait aqueux de pollen obtenu à partir d'un mélange de plantes appartenant à la famille des Pinacées et des Poacées, c'est-à-dire qu'il ne contient qu'un extrait aqueux de pollen et ne contient pas d'autre extrait de pollen tel qu'un extrait huileux.

Selon l'invention, par extrait aqueux, on entend un extrait contenant des principes actifs hydrosolubles obtenu par extraction, par exemple une hydrodistillation, une macération, une infusion, une digestion, une décoction, une percolation ou encore une lixiviation, préférentiellement une macération à basse température comprise entre 15 et 40°C, d'une matière première végétale dans un solvant aqueux, c'est-à-dire un solvant comprenant de l'eau prise seule ou avantageusement en mélange avec d'autre(s) solvant(s) tels qu'un alcool, une cétone, et/ou un tensioactif non ionique.

A titre d'exemples non limitatifs, le solvant aqueux est choisi parmi un mélange comprenant majoritairement de l'eau en combinaison avec un alcool tel que l'éthanol, une cétone telle que l'acétone, et/ou tensioactif non ionique.

Le solvant peut être ensuite éliminé partiellement ou totalement pour obtenir un extrait.

La composition selon l'invention utilisée dans le traitement de l'incontinence urinaire chez la femme comprend un extrait de pollen consistant en un extrait aqueux de pollen obtenu à partir d'un mélange de : - 45% à 90% d'extrait aqueux de pollen de *Secale céréale L.* en poids du poids total de l'extrait ; - 1% à 35% d'extrait aqueux de pollen de *Zea mays L.* en poids du poids total de l'extrait ; - 0,01% à 5% d'extrait aqueux de pollen de *Pinus sylvestris L.* en poids du poids total de l'extrait ; - 3% à 30% d'extrait aqueux de pollen de *Dactylis glomerata L.* en poids du poids total de l'extrait, un extrait aqueux de pistil de *Zea mays L.,* un extrait aqueux de graines de plante(s) appartenant au genre *Cucurbita* contenant des acides gras à une teneur de moins de 10% en poids du poids total de l'extrait, et de la vitamine E ou ses esters.

Les extraits aqueux de pollen utilisés dans la composition selon l'invention sont obtenus à partir d'un mélange de plante(s) de la famille des Poacées choisies parmi les genres Secale, Zea et Dactylis et de la famille des Pinacées choisies parmi le genre *Pinus*.

Plus particulièrement, l'extrait aqueux de pollen selon l'invention est obtenu à partir d'un mélange de plante(s) de la famille des Poacées choisies parmi les espèces *Secale céréale L.* (seigle), *Zea mays L.* (maïs), et *Dactylis glomerata L.* (dactyle), et des Pinacées choisies parmi *Pinus sylvestris L.* (pin sylvestre).

De préférence, les plantes utilisées, à partir desquelles les pollens et pistils sont obtenus, sont fraîchement récoltées.

Les pollens utilisés pour la présente invention peuvent être des pollens récoltés par des insectes (tel que du pollen d'abeille) ou récoltés par une intervention humaine. Le pollen d'abeille par exemple contient du pollen, mais aussi du nectar et de la salive d'abeille. Le pollen récolté *via* une intervention humaine est dépourvu de tels ingrédients additionnels. De préférence, lesdits pollen des présentes compositions sont obtenus seulement par une intervention humaine. Ceci permet une fois de plus une standardisation du produit final.

Après leur ramassage, les pollens et pistils peuvent être utilisés frais ou séchés, avantageusement séchés, et éventuellement débactérisés.

La composition utilisée selon l'invention comprend un extrait aqueux de pistil de *Zea mays L..*

L'extrait aqueux de pollen est obtenu à partir d'un mélange de :
- 45% à 90% d'extrait aqueux de pollen de *Secale céréale L.* en poids du poids total de l'extrait ;
- 1% à 35% d'extrait aqueux de pollen de *Zea mays L.* en poids du poids total de l'extrait ;
- 0,01% à 5% d'extrait aqueux de pollen de *Pinus sylvestris L.* en poids du poids total de l'extrait ;
- 3% à 30% d'extrait aqueux de pollen de *Dactylis glomerata L.* en poids du poids total de l'extrait.

De manière préférée, la composition utilisée selon l'invention comprend 0,1% à 10% d'extrait aqueux de pistil de *Zea mays L.* en poids du poids total de l'extrait.

A titre non limitatif d'exemple de procédé de préparation d'un extrait aqueux de pollen, et de pistil, obtenu à partir de plante(s) appartenant à la famille des Pinacées et/ou des Poacées, on peut citer un procédé comprenant les étapes successives de :
a) extraction aqueuse de pollen ;
b) extraction aqueuse de pollen et avantageusement de pistil ;
c) séchage par atomisation des extraits obtenus aux étapes a) et b) ; et
d) récupération desdits extraits de pollen et de pistil de plante(s) obtenus en c).

Le Demandeur a pu mettre en évidence que l'étape d'extraction était particulièrement sensible. Ainsi, la température de l'extraction doit être strictement inférieure à 45°C. Au-delà de cette température, un ou plusieurs pollens et éventuellement pistils détaillés ci-dessus ne sont plus présents. La qualité de la composition et/ou son efficacité ne sont donc pas assurées.

Préférentiellement, la température de l'extraction doit être inférieure à 42°C.

La durée de l'étape d'extraction, pour chacun des extraits, est préférentiellement d'au moins 6h, préférentiellement au moins 10h, plus préférentiellement encore au moins 12h.

De plus, le Demandeur a pu mettre en évidence qu'une agitation trop soutenue dégradait le ou les extraits. Ainsi, lorsque l'extraction est couplée à une agitation, celle-ci ne doit pas dépasser les 6000 tours par minute (tr/min), préférentiellement 4500 tr/min, plus préférentiellement encore 2800 tr/min.

De préférence, le procédé pour obtenir un extrait aqueux de pollen, et de pistil, selon l'invention comprend les étapes suivantes de :
a) extraction à l'eau et/ou à un mélange eau-acétone de pollens de Secale *céréale L.,* de *Zea mays L.,* de *Pinus sylvestris L.* et de *Dactylis glomerata L.,* à une température inférieure à 45°C pendant au moins 6h afin d'obtenir un premier extrait ;
b) extraction à l'eau et/ou à un mélange eau-acétone de pollens et de pistil de Zea *mays L.* à une température inférieure à 45°C pendant au moins 6h afin d'obtenir un second extrait ;
c) mélange du premier et du second extraits obtenus en a) et b) ;
d) séchage par atomisation du mélange obtenu en c) ; et
e) récupération du mélange d'extraits de pollen et de pistil obtenu en d).

Le procédé ainsi développé permet de garantir une bonne traçabilité des pollens et pistils utilisés.

La composition utilisée selon l'invention comprend également un extrait aqueux de graines de plante(s) appartenant au genre *Cucurbita*.

*Cucurbita* est un genre de plantes grimpantes ou rampantes qui appartient à la famille des Cucurbitacées. Il comprend de 12 à 14 espèces, la plupart cultivées sous le nom de courge. Les trois principales espèces domestiquées sont *Cucurbita pepo*, *Cucurbita maxima* et *Cucurbita* moschata.

L'extrait de graines de plante(s) appartenant au genre *Cucurbita* utilisé selon l'invention est un extrait aqueux contenant des acides gras à une teneur de moins de 10%, par exemple comprise entre 5 et 8%, en poids du poids total de l'extrait.

Les graines de citrouille comprennent notamment des substances actives connues pour leur effet diurétique telles que des acides gras insaturés, des stérols, des vitamines et du zinc.

Préférentiellement, la composition utilisée selon l'invention comprend un extrait aqueux de graines de *Cucurbita pepo*, *Cucurbita maxima* et *Cucurbita moschata,* prises seules ou en mélange.

Plus préférentiellement, l'extrait utilisé dans la composition selon l'invention est un extrait aqueux de graines de citrouille (*Cucurbita pepo*).

Après leur ramassage, les graines sont avantageusement séchées, puis éventuellement broyées.

Selon un mode de réalisation préféré de l'invention, l'extrait aqueux de graines de *Cucurbita* est obtenu à partir de graines de citrouille (Cucurbita *pepo*) séchées puis broyées et extraites par macération à température ambiante dans de l'eau dans un ratio compris entre 10:1 et 50:1, par exemple 20:1, 30:1 ou 40:1.

L'extrait aqueux de graines de *Cucurbita* utilisé selon l'invention est un extrait de *Cucurbita pepo* contenant des acides gras à une teneur de moins de 10%, par exemple comprise entre 5 et 8%, en poids du poids total de l'extrait. Plus préférentiellement, l'extrait aqueux de graines de *Cucurbita* utilisé selon l'invention est un extrait contenant des glucides, des acides gras à une teneur de moins de 10%, par exemple comprise entre 5 et 8%, des protéines, des fibres et du sodium.

Selon un mode de réalisation particulièrement préféré, l'extrait aqueux de graines de *Cucurbita pepo* utilisé selon l'invention comprend 52% de glucides, 6% d'acides gras, 24% de protéines, 14% de fibres, du sodium / chlorure de sodium 0,1% / 0,25%.

La composition utilisée selon l'invention comprend également de la vitamine E ou ses esters.

La vitamine E est une vitamine liposoluble recouvrant un ensemble de huit molécules organiques, quatre tocophérols (a-tocophérol, β-tocophérol, γ-tocophérol et δ-tocophérol) et quatre tocotriénols (a-tocotriénol, β-tocotriénol, γ-tocotriénol et 6-tocotriénol). La forme la plus biologiquement active est l'a-tocophérol. La forme la plus abondante dans l'alimentation est le γ-tocophérol. Ces molécules sont présentes en grande quantité dans les huiles végétales. Elles agissent, parallèlement à la vitamine C et au glutathion, essentiellement comme antioxydants contre les dérivés réactifs de l'oxygène.

Par esters de vitamine E, on entend une vitamine E stabilisée chimiquement pour éviter par exemple tout problème d'oxydation. Sous cette forme, elle n'a pas de pouvoir antioxydant. C'est en passant dans l'organisme que les tocophéryls sont hydrolysés et se transforment en tocophérols et deviennent actifs. A titre d'exemples d'ester de vitamine E, on peut citer la forme acétate ou succinate, par exemple le D-alpha-tocophéryl acétate.

Préférentiellement, la composition utilisée selon l'invention comprend :
- 5 à 40% en poids du poids total de la composition de l'extrait aqueux de pollen obtenu à partir d'un mélange de : - 45% à 90% d'extrait aqueux de pollen de *Secale céréale L.* en poids du poids total de l'extrait ; - 1% à 35% d'extrait aqueux de pollen de *Zea mays L*. en poids du poids total de l'extrait ;
- 0,01% à 5% d'extrait aqueux de pollen de *Pinus sylvestris L.* en poids du poids total de l'extrait ; et - 3% à 30% d'extrait aqueux de pollen de *Dactylis glomerata L.* en poids du poids total de l'extrait ;
- un extrait aqueux de pistil de *Zea mays L.,*
- 20 à 80% en poids du poids total de la composition de l'extrait aqueux de graines de *Cucurbita* contenant des acides gras à une teneur de moins de 10% en poids du poids total de l'extrait ; et
- 0,1 à 10% en poids du poids total de la composition de vitamine E ou ses esters.

Plus préférentiellement, la composition comprend :
- 10 à 30% en poids du poids total de la composition de l'extrait aqueux de pollen obtenu à partir d'un mélange de : - 45% à 90% d'extrait aqueux de pollen de *Secale céréale L.* en poids du poids total de l'extrait ; - 1% à 35% d'extrait aqueux de pollen de *Zea mays L.* en poids du poids total de l'extrait ;
- 0,01% à 5% d'extrait aqueux de pollen de *Pinus sylvestris L.* en poids du poids total de l'extrait ; et - 3% à 30% d'extrait aqueux de pollen de *Dactylis glomerata L*. en poids du poids total de l'extrait, par exemple 10%, 15%, 20%, 25%, 30% ;
- un extrait aqueux de pistil de *Zea mays L.,*
- 30 à 50% en poids du poids total de la composition de l'extrait aqueux de graines de *Cucurbita* contenant des acides gras à une teneur de moins de

10% en poids du poids total de l'extrait, par exemple 30%, 35%, 40%, 45%, 50% ; et - 1 à 5% en poids du poids total de la composition de vitamine E ou ses esters, par exemple 1%, 2%, 3%, 4%, 5%.

La composition utilisée selon l'invention comprend également un milieu physiologiquement acceptable proportionné à un rapport avantage/risque raisonnable, comprenant des excipients connus et couramment utilisés en phytothérapie tels que des liants, agents de désintégration, agents de charge, agents de dispersion, agents agglomérants, lubrifiants, agents mouillants, tensioactifs, émulsifiants, épaississants, agents de coulance, agents aromatisants, agents édulcorants, colorants, agents de pelliculage, stabilisants et/ou conservateurs ne contenant avantageusement pas de nanoparticules.

L'homme du métier veillera à choisir ces éventuels excipients et leur quantité de manière à ce qu'ils ne nuisent pas aux propriétés intéressantes des compositions utilisées selon l'invention.

A titre d'exemple d'excipients, on peut citer la cellulose, préférentiellement la cellulose microcristalline, et le dioxyde de silicium.

En outre, avantageusement, la composition utilisée selon l'invention est susceptible de comprendre un autre actif choisi parmi le manganèse ou encore des extraits de *Betula alba, Cerasus avium, Equisetum arvense, Phaseolus vulgaris, Achillea millefolium, Agropyron repens, Galium verum, Lavandula officinalis*, *Mentha piperita*, *Urticaria dioica.*

L'ajout d'au moins un de ces autres actifs, préférentiellement du manganèse, va potentialiser les effets de la composition utilisée selon l'invention.

La composition comprend avantageusement 0,1 à 10% en poids du poids total de la composition de l'un de ces éléments actifs pris seul ou en combinaison, par exemple de manganèse.

La composition utilisée selon l'invention est préférentiellement sous une forme adaptée pour une administration par voie orale, en une ou plusieurs formulations identiques ou différentes. Elle se présente sous une forme galénique quelconque normalement utilisée pour une administration orale et notamment sous la forme de gélule, comprimé, capsule, capsule molle, dragée, sachet, tube, flacon, chewing-gum, bille, émulsion, suspension, liquide, solution, ampoule, boisson, sirop, poudre, solide, gel mou, semi-solide.

Avantageusement, la composition est formulée sous forme de comprimé, gélule, capsule, gel mou, semi-solide, solide, liquide ou poudre.

De préférence, la dose journalière de la composition utilisée selon la présente invention est comprise entre 360 mg et 1000 mg, préférentiellement entre 600 et 1000 mg, plus préférentiellement entre 600 et 800 mg. Cette dose journalière est préférentiellement administrée en 1, 2 ou 3 prises (matin, midi et/ou soir) par exemple sous forme de 1, 2, 3, 4 ou 6 comprimés, préférentiellement en une prise, plus préférentiellement pendant une période d'au moins 2 mois, par exemple de 2, 3 ou 6 mois, préférentiellement 3 mois.

Les compositions selon l'invention sont utilisées pour le traitement de l'incontinence urinaire chez la femme.

Les symptômes de la phase de remplissage sont les symptômes ressentis pendant la phase de remplissage de la vessie sans distinction entre le jour et la nuit. Il s'agit préférentiellement des symptômes suivants :
- pollakiurie diurne c'est-à-dire une augmentation de la fréquence mictionnelle pendant la journée ;
- nycturie c'est-à-dire un besoin d'uriner réveillant le patient. La nycturie doit être différenciée de la « fréquence mictionnelle nocturne » qui correspond à la totalité des mictions nocturnes à partir du moment où le patient se couche, mais sans tenir compte de la notion de réveil. Le terme « pollakiurie nocturne » doit être réservé aux situations où l'on souhaite décrire une augmentation anormale de la fréquence mictionnelle nocturne et introduit par voie de conséquence une notion de seuil pathologique défini soit par un nombre d'événements, soit par un éventuel retentissement en termes de qualité de vie ou de gêne ;
- urgenturie ou urgence mictionnelle est un désir soudain, impérieux et fréquemment irrépressible d'uriner. Ce terme correspond à l'expression d'une envie soudaine et irrésistible d'uriner, qu'il est difficile ou impossible de différer. C'est un besoin qui est anormal par sa brutalité et son intensité. Il ne s'accompagne souvent que d'une quantité urinée modérée, voire faible. Il est différent de la progression normale du besoin qui passe d'une sensation de vessie remplie à une sensation d'envie d'uriner par vessie pleine. Le besoin physiologique normal est le signe annonciateur d'une miction qu'il est possible de différer un certain temps pour satisfaire aux commodités sociales et aux contraintes d'environnement ;

- incontinence urinaire est une fuite involontaire d'urine. Le terme d'incontinence urinaire doit être précisé selon : le mécanisme et les circonstances de survenue des fuites, leur sévérité, leur fréquence, l'existence d'éventuels facteurs favorisants, leur impact social, hygiénique ou sur la qualité de vie, l'existence d'éventuelles mesures prises pour éviter les fuites et, enfin, la notion de demande médicale de la part du patient ;
- incontinence urinaire à l'effort (IUE) est une fuite involontaire d'urine lors d'un effort physique, lors de la toux et d'éternuements ;
- incontinence urinaire par urgenturie (ou impériosité) est une fuite involontaire d'urine accompagnée ou immédiatement précédée par une urgenturie ;
- incontinence urinaire mixte (IUM) est une fuite involontaire d'urine associée à une urgenturie avec, également, fuites involontaires d'urine lors des exercices physiques, toux ou éternuements ;
- incontinence par regorgement est une fuite due au fait que la personne est incapable de vider sa vessie complètement. Il en résulte des fuites fréquentes et souvent répétées d'urine ;
- énurésie est une miction involontaire. Le terme d'énurésie nocturne qualifie l'énurésie lorsqu'elle se manifeste pendant le sommeil ;
- incontinence permanente est une fuite d'urine permanente ;
- sensibilité vésicale :
   normale : le patient décrit un besoin d'uriner progressivement croissant jusqu'à ressentir un besoin pressant ;
   augmentée : le patient décrit un besoin d'uriner très précoce et persistant ; réduite : le patient ressent l'augmentation du volume vésical mais ne ressent pas le besoin d'uriner;
   absente : le patient ne ressent aucune sensation.

Les différents symptômes reliés à la phase mictionnelle sont :
- faiblesse du jet : perception par le patient d'une diminution de la force du jet urinaire pendant la miction ;
- jet en arrosoir;
- jet haché : miction interrompue à une ou plusieurs reprises ;
- jet hésitant : retard à l'initiation de la miction ;
- miction par poussée : jet urinaire obtenu avec une poussée abdominale concomitante ;
- gouttes terminales, miction traînante : achèvement progressif et lent de la miction qui se termine par un écoulement en goutte à goutte.

Les Symptômes de la phase post-mictionnelle sont les symptômes ressentis par le patient immédiatement après la fin de la miction :
- sensation de vidange vésicale incomplète est une impression subjective que la vessie ne s'est pas totalement vidée après la miction ;
- gouttes retardataires sont une perte involontaire d'urine survenant immédiatement après la miction, le plus souvent en quittant les toilettes pour l'homme ou en se levant des toilettes pour la femme.

La dyspareunie, la sécheresse vaginale et l'incontinence urinaire sont les principaux symptômes décrits à l'occasion des rapports sexuels et peuvent être également traités avec les compositions utilisées selon l'invention.

De nombreux symptômes peuvent être exprimés par les patients, dus à un prolapsus urogénital, par exemple : sensation de pesanteur pelvienne ou de corps étranger endovaginal, nécessité de repousser une masse vaginale pour pouvoir uriner ou aller à la selle et peuvent être également traités.

La sensation de douleur à traiter avec les compositions selon l'invention peut être caractérisée quant à sa fréquence de survenue, son intensité, sa durée, l'existence d'éventuels facteurs favorisants ou encore sa localisation telle que :
- cystalgie, qui est un terme général pour définir une douleur ressentie dans la région pré- ou rétro-pubienne. La douleur augmente habituellement au fur et à mesure du remplissage vésical et peut éventuellement persister après la miction. Ce terme doit être préféré à celui de « cystite interstitielle » qui correspond à une entité pathologique définie de manière plus précise selon un ensemble de critères diagnostiques ;
- urétralgie, qui est une douleur ressentie dans l'urètre et que le patient désigne ;
- douleur vulvaire, qui est une douleur ressentie strictement au niveau vulvaire ;
- douleur vaginale, qui est une douleur ressentie à l'intérieur de la cavité vaginale au-delà du segment vulvaire ;
- douleur scrotale, dont la description ne présage pas d'une localisation plus spécifique au cordon, au testicule, à l'épididyme, ou encore cutanée ;
- douleur périnéale, qui est une est une douleur ressentie, chez la femme, entre la fourchette vulvaire et l'anus et, chez l'homme, entre le scrotum et l'anus ;
- douleur pelvienne, qui est une douleur moins bien définie quant à sa localisation précise, plus diffuse et sans lien direct avec le cycle de remplissage-miction de la vessie.

Les compositions selon l'invention sont utilisées dans le traitement de l'incontinence urinaire chez la femme. Elles sont plus particulièrement utilisées pour le traitement de l'incontinence urinaire à l'effort (IUE), l'incontinence urinaire par urgenturie (ou impériosité), l'incontinence urinaire mixte (IUM), l'incontinence par regorgement, l'énurésie et/ou l'incontinence permanente, encore plus préférentiellement pour le traitement et/ou la prévention de l'incontinence urinaire à l'effort (IUE), l'incontinence urinaire par urgenturie (ou impériosité) et l'incontinence urinaire mixte (IUM), dont notamment la réduction de la fréquence mictionnelle nocturne induisant un réveil.

Les compositions selon l'invention comprenant un extrait de pollen consistant en un extrait aqueux de pollen obtenu à partir d'un mélange de : - 45% à 90% d'extrait aqueux de pollen de *Secale céréale L.* en poids du poids total de l'extrait ; - 1% à 35% d'extrait aqueux de pollen de *Zea mays L.* en poids du poids total de l'extrait ; - 0,01% à 5% d'extrait aqueux de pollen de *Pinus sylvestris L.* en poids du poids total de l'extrait ; et 3% à 30% d'extrait aqueux de pollen de *Dactylis glomerata L.* en poids du poids total de l'extrait, un extrait aqueux de pistil de *Zea mays L.,* un extrait aqueux de graines de plante(s) appartenant au genre *Cucurbita* contenant des acides gras à une teneur de moins de 10% en poids du poids total de l'extrait, et de la vitamine E ou ses esters sont utilisées dans le traitement de l'incontinence urinaire, en particulier de l'incontinence urinaire à l'effort (IUE), l'incontinence urinaire par urgenturie (ou impériosité) et l'incontinence urinaire mixte (IUM), dont notamment la réduction de la fréquence mictionnelle nocturne induisant un réveil.

Il est connu que les fonctions du bas appareil urinaire, pour stocker et évacuer périodiquement l'urine, dépendent de l'activité des muscles lisses et striés dans la vessie, l'urètre et le sphincter externe de l'urètre.

Pendant le stockage de l'urine, la sortie est fermée et le muscle lisse de la vessie est en repos. Lorsque le volume de la vessie atteint le seuil de miction, l'activation d'un centre pontique activateur de la miction induit une contraction de la vessie et une relaxation réciproque de l'urètre, entraînant la vidange de la vessie.

Au cours de la vidange, les nerfs parasympathiques sacrés (pelviens) exercent une action excitatrice (cholinergique et purinergique) sur la vessie via les nerfs pelviens et une action inhibitrice (nitrergique) sur l'urètre. Ces systèmes périphériques sont intégrés par une régulation excitatrice et inhibitrice au niveau de la moelle épinière et du cerveau. Les lésions ou les maladies du système nerveux, ainsi que les médicaments et les troubles des organes périphériques, peuvent provoquer un dysfonctionnement des voies urinaires inférieures.

Dans le cas d'une vessie hyperactive (OAB), des cibles thérapeutiques pour faciliter le stockage des urines peuvent être trouvées au niveau de l'urothélium, du muscle détrusor, des voies autonome et afférente, de la moelle épinière et du cerveau.

L'urothélium apparaît avoir des propriétés sensorielles et de signalisation spécialisée, incluant : (1) l'expression de récepteurs nicotinique, muscarinique, de tachykinine, adrénergique, de bradykinine et de potentiel récepteur transitoire (TRP), (2) une association physique étroite avec des nerfs afférents, et (3) la capacité à libérer des molécules chimiques telles que l'adénosine triphosphate (ATP), l'acétylcholine et l'oxyde nitrique.

L'augmentation de l'expression et/ou de la sensibilité à ces molécules sensorielles urothéliales qui conduisent à une sensibilité afférente peut être responsables de l'OAB. Le ciblage des voies afférentes et/ou des muscles lisses de la vessie en modulant l'activité des récepteurs (par exemple neurokinine, ATP ou β3-adrénergique) et des canaux ioniques (par exemple TRPV1 ou K) pourrait être efficace pour supprimer l'OAB.

Dans le cas d'une incontinence urinaire à l'effort (stress), les pharmacothérapies ciblant le réflexe de continence de l'urètre médié par voie neuronale pendant des conditions d'effort telles que les éternuements ou la toux pourraient être efficaces pour augmenter la résistance de sortie. Les cibles thérapeutiques comprennent les récepteurs adrénergiques et sérotoninergiques dans la moelle épinière ainsi que les récepteurs adrénergiques du sphincter urétral, qui peuvent respectivement améliorer l'activité du réflexe urétral pendant les conditions de stress et augmenter la pression urétrale de base.

Ainsi, les compositions selon l'invention utilisées dans le traitement et/ou la prévention de l'incontinence urinaire pourraient avoir une action au niveau du système nerveux, en particulier des voies afférentes et du muscle lisse, en agissant par exemple sur une ou plusieurs de ces cibles moléculaires dans l'urothélium, l'urètre et la vessie.

Les compositions sont également utilisées pour le traitement et/ou la prévention des syndromes douloureux génito-urinaires et syndromes cliniques évocateurs de dysfonctionnement du bas appareil urinaire.

Les syndromes sont des associations de différents symptômes, mais ils ne sont pas nécessairement pathognomoniques d'un état pathologique. Le terme de « syndrome » ne peut être employé que s'il existe plusieurs symptômes chez un même patient. Les différents syndromes traités correspondent à des anomalies fonctionnelles pour lesquelles aucune cause précise n'a été identifiée, étant bien entendu que des moyens usuels d'investigations ont éliminé une pathologie organique locale évidente comme, par exemple, infection, tumeur, trouble hydroélectrolytique.

Les syndromes douloureux génito-urinaires sont tous chroniques par nature. La douleur, qui est la plainte prédominante, peut être associée à d'autres symptômes concernant le bas appareil urinaire, la sphère génitosexuelle ou encore le tractus digestif :
- le syndrome douloureux vésical est une douleur sus-pubienne majorée lors du remplissage vésical, associé à une pollakiurie diurne ou nocturne en l'absence d'infection urinaire ou de pathologie tissulaire vésicale ;
- le syndrome douloureux urétral est une douleur urétrale récurrente survenant préférentiellement lors de la miction en l'absence d'infection urinaire ou de pathologie tissulaire vésicale ;
- le syndrome douloureux vulvaire est une douleur vulvaire permanente ou intermittente pouvant être variable en fonction du cycle mictionnel ou être associée à des symptômes évocateurs d'un dysfonctionnement du bas appareil urinaire ou à des troubles sexuels ;
- le syndrome douloureux vaginal est une douleur vaginale permanente ou intermittente associée à des symptômes évocateurs d'un dysfonctionnement du bas appareil urinaire ou à des troubles sexuels, et ce en l'absence d'infection vaginale prouvée ou de toute autre pathologie vaginale évidente ;
- le syndrome douloureux scrotal est une douleur scrotale permanente ou intermittente associée à des symptômes évocateurs d'un dysfonctionnement du bas appareil urinaire ou à des troubles sexuels, et ce en l'absence d'épididymite ou de toute autre pathologie locale ;
- le syndrome douloureux périnéal est une douleur périnéale permanente ou intermittente associée à des symptômes évocateurs d'un dysfonctionnement du bas appareil urinaire ou à des troubles sexuels, et ce en l'absence d'infection prouvée ou de tout autre pathologie périnéale évidente ;
- le syndrome douloureux pelvien est une douleur pelvienne permanente ou intermittente associée à des symptômes évocateurs d'un dysfonctionnement du bas appareil urinaire, de l'appareil digestif ou à des troubles sexuels, et ce en l'absence d'infection prouvée ou de tout autre état pathologique évident.

En pratique clinique, une approche diagnostique empirique est fréquemment utilisée pour décider du mode de prise en charge d'un patient souffrant de symptômes évocateurs d'un dysfonctionnement du bas appareil urinaire :
- le « syndrome clinique d'hyperactivité vésicale » ou « syndrome urgenturiepollakiurie » est défini par la survenue d'urgenturies avec ou sans incontinence urinaire, habituellement associées à une pollakiurie ou une nycturie. Ce syndrome est évocateur d'une hyperactivité détrusorienne mise en évidence par un examen urodynamique, mais non spécifique car pouvant également être dû à d'autres types de dysfonctionnement du bas appareil urinaire. Par ailleurs, le terme de « syndrome clinique d'hyperactivité vésicale » suppose qu'il n'y ait pas d'infection urinaire ou une pathologie locale organique évidente (tumeur, etc.) ;
- le « syndrome dysurique » évocateur d'obstruction sous-vésicale doit être utilisé chez un homme présentant de manière prédominante des troubles mictionnels, et ce en l'absence d'infection urinaire ou d'une pathologie locale évidente. Chez la femme, un syndrome dysurique doit faire évoquer plutôt une hypoactivité vésicale qu'une obstruction sous-vésicale.

### Exemples

La présente invention va maintenant être illustrée au moyen des exemples suivants :

**Exemple 1 : Comprimé (Tableau 1)**

| Ingrédients | Quantité mg/comprimé |
|---|---|
| Extrait aqueux de pollen contenant en poids du poids total de l'extrait : | 120-200 |
| 70-75% de *Secale cereale L.* Pollen | |
| 15-20% de *Zea mays* L. Pollen | |
| 0,05-1,00 de *Pinus sylvestris L.* Pollen | |
| 5-10% de *Dactylis glomerata L.* Pollen | |
| et 1-4% d'un Extrait aqueux de pistil de *Zea mays L.* | |
| Extrait aqueux de graines de *Cucurbita* contenant moins de 10% d'acides gras | 200-400 |
| Ester de Vitamine E | 16-30 |
| Cellulose microcristalline | 100-300 |
| Dioxyde de silicium | 1-10 |
| Agent de revêtement | 10-50 |

**Exemple 2 : Gélule (Tableau 2)**

| Ingrédients | Quantité mg/gélule |
|---|---|
| Extrait aqueux de pollen contenant en poids du poids total de l'extrait : | 120-200 |
| 70-75% de *Secale cereale L*. Pollen | |
| 15-20% de Zea *mays L*. Pollen | |
| 0,05-1,00 de *Pinus sylvestris L.* Pollen | |
| 5-10% de *Dactylis glomerata L*. Pollen | |
| et avantageusement 0.6-2% d'un Extrait aqueux de pistil de Zea *mays L.* | |
| Extrait aqueux de graines de *Cucurbita* contenant moins de 10% d'acides gras | 100-200 |
| Ester de Vitamine E | 8-20 |
| Cellulose microcristalline | 100-300 |
| Dioxyde de silicium | 1-10 |
| Lubrifiant | 1-5 |
| Agent anti-agglomérant | 100-400 |

**Exemple 3 : Capsule molle (Tableau 3)**

| Ingrédients | Quantité mg/capsule |
|---|---|
| Extrait aqueux de pollen contenant en poids du poids total de l'extrait : | 120-200 |
| 70-75% de *Secale cereale L.* Pollen | |
| 15-20% de *Zea* mays *L.* Pollen | |
| 0,05-1,00 de *Pinus sylvestris L.* Pollen | |
| 5-10% de *Dactylis glomerata L.* Pollen | |
| et avantageusement 1-4% d'un Extrait aqueux de pistil de *Zea mays L.* | |
| Extrait aqueux de graines de *Cucurbita* contenant moins de 10% d'acides gras | 200-400 |
| Ester de Vitamine E | 16-30 |
| Huile végétale | 100-500 |

**Exemple 4 : Gélule (Tableau 4)**

| Ingrédients | Quantité mg/gélule |
|---|---|
| Extrait aqueux de pollen contenant en poids du poids total de l'extrait : | 200-400 |
| 70-75% de *Secale cereale L.* Pollen | |
| 15-20% de *Zea mays L.* Pollen | |
| 0,05-1,00 de *Pinus sylvestris L.* Pollen | |
| 5-10% de *Dactylis glomerata L*. Pollen | |
| et avantageusement 0,6-4% d'un Extrait aqueux de pistil de *Zea mays L.* | |
| Extrait aqueux de graines de *Cucurbita* contenant moins de 10% d'acides gras | 100-200 |
| Ester de Vitamine E | 8-20 |
| Cellulose microcristalline | 100-300 |
| Dioxyde de silicium | 1-10 |
| Lubrifiant | 1-5 |
| Agent anti-agglomérant | 100-400 |

Les compositions selon l'invention comprenant un extrait de pollen contenant (et préférentiellement consistant en) un extrait aqueux de pollen, un extrait aqueux de graines de *Cucurbita* contenant moins de 10% d'acides gras et de la vitamine E ou ses esters, et avantageusement en outre un extrait aqueux de pistil de *Zea mays L.,* sont formulées suivant les techniques communément connues et utilisées par l'homme du métier.

### Exemple 5 : Etude d'activité sur l'incontinence urinaire chez la femme

Une étude a été réalisée sur 160 patientes âgées entre 18 et 75 ans (60,35 d'âge moyen) souffrant de SBAU, et plus particulièrement d'incontinence urinaire.

Le taux d'abandon de l'étude est de 21,875%, en raison de causes telles que les conseils du médecin de famille, le changement de traitement, les effets indésirables, et l'absence de cause précise et la perte de suivi (9 patientes).

Parmi les patientes ayant suivi l'étude, l'observance du traitement a été très satisfaisante ; les comprimés ont été correctement pris dans le temps et dans la forme.

Les résultats ont été obtenus après administration 1 fois par jour pendant au moins 2 mois (et jusqu'à 3 mois) avec la composition C (composition selon l'exemple 1) et comparés avec l'administration dans les mêmes conditions de soit :
- une composition A comprenant 120-200 mg d'un extrait aqueux de pollen P2 et 5-10 mg d'un extrait huileux de pollen EA5 (= GRAMINEX^{®}), 100-300 mg d'un extrait aqueux de graines de citrouille et 16-30 mg de D-alpha-tocophéryl acétate ;
- une composition B comprenant 120-200 mg d'un extrait aqueux de pollen P2 et 5-10 mg d'un extrait huileux de pollen EA5 (= GRAMINEX^{®}) ; ou
- un placébo (comprimé comprenant de la microcristaline cellulose, isomalt, magnésium stéarate, silicone dioxide, et un agent de revêtement (Aqua Polish D Grey 089.45 MS)).

L'efficacité des différentes compositions a été évaluée avec des questionnaires validés, à savoir les échelles de SANDVIK, MHU (Mesure du Handicap Urinaire) et ICIQ (International Consultation on Incontinence Questionnaire).

Le score de sévérité développé par SANDVIK (Incontinence Severity Index ; Sandvik H, Hundskaar S et al. Validation of a severity index in female urinary incontinence and its implementation in a epidemiological survey. J Epidemiol Community Health 1993;47:497-99) permet de classer la sévérité de l'incontinence en quatre stades (minime, modérée, sévère, très sévère).

L'échelle MHU (Mesure du Handicap Urinaire) publiée par Amarenco en 1992 (Amarenco G, Kerdraon J, Perrigot M. Échelle d'évaluation du handicap pelvien : mesure du handicap urinaire (MHU). In: Rééducation vésicosphinctérienne et anorectale. Edité par Pelissier J, Costa P, Lopez S, Mares P, Masson Ed, 1992, 498-504) permet d'obtenir des mesures quantitatives de différents symptômes urinaires. Ce questionnaire est utilisable quel que soit le type d'incontinence urinaire. Il comporte 7 questions avec une échelle de réponse de 0 à 4. Il a été modifié pour l'incontinence urinaire féminine non neurologique en supprimant la question sur« autre incontinence » et en la remplaçant par une question sur la fréquence de l'incontinence d'effort. Il aboutit à un score de 0 à 28 avec quatre sous-scores : impériosité (0 à 8), effort (0 à 8), pollakiurie (0 à 8) et dysurie (0 à 4).

L'échelle ICIQ (International Consultation on Incontinence Questionnaire ; Avery K, Donovan J, Peters TJ et al. (2004) International Consultation on Incontinence Questionnaire (ICIQ): a brief and robust measure for evaluating the symptoms and impact of urinary incontinence. Neurourol Urodyn 2004;23:322-30.) est un des plus récents questionnaires développés chez la femme pour évaluer l'incontinence urinaire (d'effort, par urgenturie ou mixte). Il comporte seulement quatre items. Parmi eux, trois évaluent l'incontinence en tant que symptôme (fréquence, volume et circonstances des fuites), le quatrième item mesurant l'impact sur la qualité de vie sous forme d'une échelle visuelle analogique.

En particulier, les résultats obtenus avant traitement (J0), après 60 jours de traitement (J60) et après 90 jours de traitement (J90) ont été mesurés avec ces différentes échelles et sont compilés dans le Tableau 5 ci-après. Les résultats ainsi obtenus sont également illustrés par les Figures 1 à 3.

Dans ces Figures 1 à 3 ainsi que dans les Figures 4 à 9, il est fait mention des références suivantes :
a-c. Comparaisons deux à deux. Au sein d'un même groupe, différentes lettres minuscules indiquent des différences statistiquement significatives entre chaque temps (correction de Bonferroni).
A-B. Comparaisons deux à deux. Pour un même temps donné, des lettres majuscules différentes indiquent des différences statistiquement significatives entre les groupes (correction de Bonferroni).

**Tableau 5 :**

| SCORE TOTAL SANDVIK | | | | | | |
|---|---|---|---|---|---|---|
| | moyenne (ecart type) | | moyenne (ecart type) | | moyenne (ecart type) | |
| temps | 0 mois | | 2 mois | | 3 mois | |
| A | 6,35 | 2,36 | 3,68 | 1,94 | 2,87 | 2,64 |
| placebo | 5,91 | 2,14 | 4,03 | 2,5 | 3,53 | 2,73 |
| B | 5,91 | 2,78 | 3,66 | 2,87 | 3,06 | 2,96 |
| placebo | 5,91 | 2,14 | 4,03 | 2,5 | 3,53 | 2,73 |
| C | 6,08 | 2,32 | 3,25 | 2,88 | 2,33 | 2,3 |
| placebo | 5,91 | 2,14 | 4,03 | 2,5 | 3,53 | 2,73 |

| SCORE TOTAL MHU | | | | | | |
|---|---|---|---|---|---|---|
| | moyenne (ecart type) | | moyenne (ecart type) | | moyenne (ecart type) | |
| A | 9,92 | 3,73 | 4,74 | 3,49 | 4,26 | 3,7 |
| placebo | 8,13 | 3,48 | 5,34 | 3,34 | 3,84 | 3,15 |
| B | 9,21 | 3,54 | 5,74 | 3,82 | 5 | 3,89 |
| placebo | 8,13 | 3,48 | 5,34 | 3,34 | 3,84 | 3,15 |
| C | 9,76 | 4,04 | 3,11 | 3,81 | 2,45 | 3,19 |
| placebo | 8,13 | 3,48 | 5,34 | 3,34 | 3,84 | 3,15 |

| SCORE TOTAL ICIQ | | | | | | |
|---|---|---|---|---|---|---|
| | moyenne (ecart type) | | moyenne (ecart type) | | moyenne (ecart type) | |
| A | 10,24 | 3,92 | 5,66 | 3,77 | 4,84 | 3,57 |
| placebo | 9,71 | 3,14 | 7,13 | 4,11 | 6,53 | 4,71 |
| B | 9,87 | 4,77 | 6,67 | 4,13 | 6,41 | 4,98 |
| placebo | 9,71 | 3,14 | 7,13 | 4,11 | 6,53 | 4,71 |
| C | 9,76 | 4,56 | 3,92 | 4,44 | 3,5 | 3,81 |
| placebo | 9,71 | 3,14 | 7,13 | 4,11 | 6,53 | 4,71 |

Les résultats de l'étude avec les trois questionnaires analysés (SANDVIK, MHU et ICIQ) montrent que les trois groupes de patientes ayant reçu respectivement les compositions A, B ou C présentent une amélioration statistiquement significative par rapport au groupe placebo.

Parmi les trois compositions administrées, il apparaît que la composition C (suivant l'exemple 1 selon l'invention) est celle qui a apporté le plus d'amélioration chez les patientes, suivi du groupe de traitement avec la composition A puis enfin avec la composition B.

Par exemple, après 60 jours de traitement avec la composition C selon l'invention, on remarque une diminution moyenne de la sévérité et/ou des symptômes urinaires de l'ordre de 46,55% (score SANDVIK), 68,14% (score MHU) et 59,44% (score ICIQ) chez les patientes traitées.

Par comparaison, les diminutions moyennes obtenues respectivement avec les compositions A et B sont seulement de 42,05% et 38,07% (scores SANDVIK), 52,22% et 37,68% (scores MHU) et 44,73% et 32,42% (scores ICIQ).

Après 90 jours de traitement, les diminutions moyennes avec la composition C selon l'invention sont respectivement de 61,68% (SANDVIK), 74,9% (MHU) et 64,14% (ICIQ).

Par comparaison, les diminutions moyennes obtenues respectivement avec les compositions A et B sont seulement de 54,8% et 48,22% (scores SANDVIK), 57,07% et 45,71% (scores MHU) et 52,73% et 35,06% (scores ICIQ).

Ces résultats sont cohérents avec la diminution de la gravité de l'incontinence urinaire, initialement classée comme très grave, grave et modérée et définie à la fin du traitement avec un pourcentage significatif de cas légers et même de patientes qui ne sont plus incontinentes et sont totalement rétablies.

Ces résultats démontrent l'efficacité du traitement plus particulièrement avec la composition C (exemple 1 selon l'invention), avec une efficacité maximale à 60 jours, l'effet positif étant maintenu 90 jours après le début (lors de la dernière visite).

Plus particulièrement, les résultats obtenus par type d'incontinence pour chacune des compositions A à C testées sont respectivement illustrés par les Figures 4 à 6 avec l'échelle ICIQ, par les Figures 7 à 9 avec l'échelle MHU et par la Figure 10 avec l'échelle SANDVIK pour la composition C selon l'invention.

D'après les résultats obtenus, selon un mode de réalisation avantageux de l'invention, l'incontinence urinaire par urgenturie (ou impériosité) et l'incontinence urinaire mixte sont plus particulièrement améliorées de manière statistiquement significative à 60 jours.

Par exemple, tel qu'illustré par la Figure 6, après 60 jours de traitement avec la composition C selon l'invention, on remarque une diminution moyenne de la sévérité et/ou des symptômes urinaires avec le score ICIQ de 36,87% pour l'incontinence urinaire à l'effort, de 62,68% pour l'incontinence urinaire par urgenturie et de 75,69% pour l'incontinence urinaire mixte, les scores étant respectivement de 40,28%, 64,49% et 80,4% après 90 jours de traitement.

De même, tel qu'illustré par la Figure 9, après 60 jours de traitement avec la composition C selon l'invention, on remarque une diminution moyenne de la sévérité et/ou des symptômes urinaires avec le score MHU de 70,66% pour l'incontinence urinaire à l'effort, de 64,15% pour l'incontinence urinaire par urgenturie et de 72,67% pour l'incontinence urinaire mixte, les scores étant respectivement de 82,66%, 70,53% et 75,47% après 90 jours de traitement.

Enfin, tel qu'illustré par la Figure 10, d'après le score obtenu avec l'échelle de SANDVIK, on peut remarquer une amélioration significative de la sévérité de l'incontinence urinaire chez les patientes traitées avec la composition C selon l'invention : on passe par exemple de 39,4% de cas très sévère (2,6%) et sévère (36,8%) à seulement 8,4% (4,2% + 4,2%) à J60 avec l'apparition de 54,2% de cas légère et même 4,2% de patientes totalement rétablies, voire même respectivement 45,8% (légère) et 20,8% (totalement rétablies) à J90.

### Exemple 6 : Etude de sécurité et d'efficacité d'une composition selon l'invention chez des femmes présentant une incontinence urinaire par urgenturie (ou impériosité) ou une incontinence urinaire mixte (essai clinique randomisé, en double aveugle et contrôlé par placebo)

Le but de cette étude est d'identifier la réponse d'une composition selon l'invention chez des femmes ayant un diagnostic de symptômes de vessie hyperactive (OAB) présentant une incontinence urinaire par urgenturie (ou impériosité) ou une incontinence urinaire mixte.

Dans cette étude, 52 femmes ont été divisées en deux groupes. Un a été traité avec la composition selon l'invention (selon l'exemple 1 = PSCEP dans les Figures 11-13) (n = 26) et un a été traité avec placebo (n = 26). L'efficacité été évaluée à l'aide de questionnaires validés, à savoir les échelles de SANDVIK, MHU (Mesure du Handicap Urinaire) et ICIQ-UI SF (International Consultation on Incontinence Questionnaire-Urinary Incontinence Short Form).

Aucun effet secondaire n'a été observé dans aucun des groupes.

### Résultats

Aucune différence au départ en termes de caractéristiques démographiques et d'incontinence entre les 2 groupes n'est à signaler.

Tel qu'illustré par la Figure 11, après 90 jours, avec le ICIQ-UI SF, une amélioration significative a été observée dans le groupe traité avec la composition selon l'invention « PSCEP» (p <0,001) par rapport à la ligne de base et en comparaison avec le groupe placebo, une amélioration significative a également été montrée (p = 0,023).

Tel qu'illustré par la Figure 12, avec le MHU, une amélioration significative a été montrée dans le groupe traité avec la composition selon l'invention « PSCEP » (p <0,001) par rapport à la ligne de base. Par rapport au groupe placebo, une amélioration significative après 90 jours (p = 0,009) a été observée chez les femmes traitées avec la composition selon l'invention « PSCEP ».

Tel qu'illustré par la Figure 13, avec l'indice Sandvik, il y a eu une amélioration significative après 90 jours de traitement dans le groupe avec la composition selon l'invention « PSCEP » par rapport à la ligne de base (p <0,001) ; cependant, il n'apparaît pas y avoir de différence statistiquement significative par rapport au groupe placebo (p = 0,23).

### Exemple 7 : Etude de sécurité et d'efficacité d'une composition selon l'invention chez des femmes présentant une incontinence urinaire à l'effort (essai clinique randomisé, en double aveugle et contrôlé par placebo)

Le but de cette étude est d'identifier la réponse d'une composition selon l'invention chez des femmes ayant un diagnostic d'incontinence à l'effort.

Dans cette étude, 24 femmes ont été divisées en deux groupes. Un a été traité avec la composition selon l'invention (selon l'exemple 1 = PSCEP dans les Figures 14-16) (n = 12) et un a été traité avec placebo (n = 12). L'efficacité été évaluée à l'aide de questionnaires validés, à savoir les échelles de SANDVIK, MHU (Mesure du Handicap Urinaire) et ICIQ-UI SF (International Consultation on Incontinence Questionnaire-Urinary Incontinence Short Form).

Aucun effet secondaire n'a été observé dans aucun des groupes.

### Résultats

Tel qu'illustré par la Figure 14, avec le ICIQ-UI SF, une amélioration significative dans le groupe traité avec la composition selon l'invention « PSCEP » par rapport au groupe placebo a été observée après 3 mois (p = 0,048).

Tel qu'illustré par la Figure 15, avec le MHU, une amélioration significative dans le groupe traité avec la composition selon l'invention « PSCEP » a été observée (p <0,001) par rapport à la ligne de base, et une amélioration significative après 3 mois de traitement par rapport au groupe placebo (p = 0,021).

Tel qu'illustré par la Figure 16, avec l'indice Sandvik, il y a eu une amélioration significative après 3 mois de traitement dans le groupe traité avec la composition selon l'invention « PSCEP » par rapport à l'inclusion (p = 0,006). Le groupe placebo n'a montré aucun changement significatif à trois mois (p = 0,102). Cependant, il n'apparaît pas y avoir de différence statistiquement significative par rapport au groupe actif (p = 0,225), probablement en raison du nombre « insuffisant » de patients.

### Exemple 8 : Etude d'efficacité sur la fréquence mictionnelle nocturne d'une composition selon l'invention

Dans cette étude, 26 femmes ayant un diagnostic de symptômes de vessie hyperactive (OAB) présentant une incontinence urinaire par urgenturie (ou impériosité) ou une incontinence urinaire mixte ont été traités avec la composition selon l'invention (selon l'exemple 1) (n = 26)

La « fréquence mictionnelle nocturne» (ou nycturie) correspond à la totalité des mictions nocturnes à partir du moment où la patiente se couche en tenant compte de la notion de réveil. Les patientes incluses dans l'étude ont été classées dans les catégories suivantes :
0 ou 1 miction par nuit (Inuit) ;
2 mictions/nuit ;
3-4 mictions/nuit ;
5-6 mictions/nuit ; ou
plus de 6 mictions/nuit

### Résultats

Tel qu'illustré par la Figure 17, la composition selon l'invention (selon l'exemple 1) réduit significativement la fréquence mictionnelle nocturne des patientes présentant une incontinence urinaire par urgenturie (ou impériosité) ou une incontinence urinaire mixte :
avec la composition selon l'invention, le pourcentage de patientes qui présentaient plus de 6 mictions par nuit au début de l'étude (inclusion), disparaît après 90 jours de traitement : plus aucune patiente ne se retrouve donc dans cette catégorie avec plus de 6 mictions/nuit ;
le pourcentage de celles qui présentaient de 3-4 mictions par nuit, est réduit de 50%;
le pourcentage de celles qui présentaient 2 mictions par nuit est réduit de 55% ;
en conséquence, on retrouve alors dans le groupe de patientes qui présentaient 0-1 miction par nuit, 3,3 fois plus de patientes.

### Exemple 9 : Etude de l'activité de la composition selon l'invention sur le récepteur muscarinique M3

L'acétylcholine (Ach) est un neurotransmetteur libéré par les cellules nerveuses pour envoyer des signaux à d'autres cellules, *i.*e. neurones, cellules musculaires et cellules glandulaires. L'acétylcholine exerce ses effets en se liant à des récepteurs situés à la surface de ces cellules et en les activant. Il existe deux classes principales de récepteurs d'acétylcholine, nicotinique et muscarinique, qui sont présents dans différents tissus. Dans la vessie, l'action de l'acétylcholine dépend de la présence de récepteurs muscariniques qui sont situés sur le muscle lisse du détrusor. Parmi les 5 types de récepteurs muscariniques appelés M1, M2, M3, M4 et M5, M2 et M3 sont présents dans la vessie. M2 prédomine en nombre et M3 est principalement le médiateur des réponses contractiles directes à l'acétylcholine. Dans une vessie saine, pour qu'elle se contracte, l'acétylcholine est libérée des nerfs cholinergiques et active le récepteur M3 dans le muscle du détrusor, ce qui entraîne la vidange de l'urine lorsqu'elle est accompagnée d'une relaxation de la sortie.

Cependant, dans la vessie hyperactive (VHA), soit une plus grande quantité d'acétylcholine est libérée pendant la distension normale de la vessie, soit les récepteurs de l'acétylcholine dans le muscle du détrusor sont plus sensibles. Cela entraîne une hyper contractilité de la vessie. Dans la vessie hyperactive, les traitements pharmacologiques sont des traitements directs, par exemple, l'administration d'antagonistes des récepteurs muscariniques agissant directement sur les récepteurs M3.

Le but de l'étude est de tester l'activité d'une composition selon l'invention (selon l'exemple 1) sur le récepteur muscarinique M3 en utilisant des cellules HEK293 qui expriment le récepteur de manière endogène (Crittenden C. et al., 2012, poster).

La composition selon l'invention a été testée seule, ainsi que chaque ingrédient actif de la composition (selon l'exemple 1) pris indépendamment.

Les composés ont été testés en utilisant une technologie de référence pour le profilage des composés Gq GPCR : appelée FLIPR Tetra, en utilisant le kit Screen Quest^{™} Fluo-8^{®} No Wash Calcium Assay Kit (Euromedex).

Le carbachol a été utilisé comme agoniste de référence et l'augmentation intracellulaire du calcium a été évaluée par la technologie FLIPR Tetra, comme lecture de l'activation de la GPCR.

La robustesse de l'essai a été confirmée par la détermination de l'affinité du carbachol dans les différents essais. Des courbes dose-réponse du carbachol (10 concentrations) ont été réalisées lors d'expériences préliminaires et la valeur calculée de la EC50 du carbachol a été utilisée pour tester l'activité antagoniste de la composition selon l'invention et de chaque ingrédient actif pris indépendamment.

Le 4-DAMP a été utilisé comme antagoniste de référence du récepteur M3. La valeur d'IC50 mesurée est de 0,26 nM ou 0,00183 µg/ml comme décrit précédemment (Dörje et al., 1991).

Dans chaque essai, des témoins positifs (carbachol) et négatifs (4-DAMP ou HBSS) ont été inclus pour normaliser les données, évaluer la robustesse de l'essai et surveiller l'efficacité.

Les cellules HEK293 ont été ensemencées sur un fond plat transparent en Poly-D-Lysine, 96 plaques de 96 puits noires incubées pendant la nuit à 37°C avec 5% de CO₂. Le lendemain, les plaques ont été lavées, le milieu remplacé par 50 µL tampon physiologique (HBSS) et les cellules chargées de colorant calcique Fluo-8 NW (Molecular Devices). Le colorant Fluo-8 NW a été utilisé conformément aux instructions du fabricant : solubilisé dans du DMSO, puis mélangé dans du tampon de dosage (1X), composé de 10 mL de 10X Pluronic^{®} F127 Plus (Molecular Devices) dans 90 mL de tampon HBSS.

Une solution de colorant (110 µL) a été ajoutée aux plaques des cellules et incubée à température ambiante pendant 1,5 heure. La fluorescence a ensuite été mesurée à l'aide du lecteur de plaques FLIPR Tetra. Pendant les 10 premières secondes, la ligne de base a été lue. Après 10 secondes, 20 µL de différents composés ont été testé dans 10 doses différentes et ont été ajoutés dans les puits et la fluorescence a été contrôlée pendant 3 minutes à Ex/Em = 490/525 nm.

Ensuite, 10 secondes d'une deuxième ligne de base ont été mesurées, puis 20 µL de carbachol (agoniste de référence) a été ajouté aux puits et la fluorescence a été contrôlée pendant 3 minutes à Ex/Em = 490/525 nm.

### Résultats

Les courbes dose-réponse de la composition selon l'invention (selon l'exemple 1), de chacun des ingrédients actifs testés indépendamment et de l'antagoniste de référence ont été normalisées. Les données de IC50 sont présentées dans le tableau ci-dessous et illustrés par la Figure 18 :

**Tableau 6 :**

| **Composés** | **IC50 µg/ml** |
|---|---|
| 4-DAMP | 0,00183 |
| Composition selon l'invention (selon l'exemple 1) | 0,6 |
| Extrait aqueux de pollen + extrait aqueux de pistil (comme dans l'exemple 1) | 96 |
| Extrait aqueux de graines de *Cucurbita* (comme dans l'exemple 1) | 1,19 |
| Vitamine E (comme dans l'exemple 1) | >1 |

Les résultats mettent en évidence que la valeur d'IC50 est sensiblement plus faible pour la composition selon l'invention que la valeur d'IC50 de chacun des ingrédients actifs testés indépendamment.

Ainsi, d'après ces résultats, on observe une activité antagoniste significative sur M3 avec la composition selon l'invention, démontrant une synergie entre les différents ingrédients actifs de la composition selon l'invention alors que chacun des ingrédients actifs pris indépendamment n'a pas (ou très peu) d'activité antagoniste sur M3 ; ces résultats d'activité sur M3 donnent également une éventuelle indication du mécanisme d'action potentiel.

## Revendications

1. Composition comprenant :
- un extrait de pollen consistant en un extrait aqueux de pollen obtenu à partir d'un mélange de :
- 45% à 90% d'extrait aqueux de pollen de Secale céréale L. en poids du poids total de l'extrait ;
- 1% à 35% d'extrait aqueux de pollen de Zea *mays* L. en poids du poids total de l'extrait ;
- 0,01% à 5% d'extrait aqueux de pollen de *Pinus sylvestris* L. en poids du poids total de l'extrait ; et
- 3% à 30% d'extrait aqueux de pollen de *Dactylis glomerata* L. en poids du poids total de l'extrait,
- un extrait aqueux de pistil de *Zea mays L.,*
- un extrait aqueux de graines de plante(s) appartenant au genre *Cucurbita* contenant des acides gras à une teneur de moins de 10% en poids du poids total de l'extrait, et
- de la vitamine E ou ses esters,
pour son utilisation dans le traitement de l'incontinence urinaire chez la femme.

2. Composition pour son utilisation selon la revendication 1, **caractérisée en ce que** l'extrait aqueux de graines de *Cucurbita* contient des acides gras à une teneur comprise entre 5 et 8% en poids du poids total de l'extrait.

3. Composition pour son utilisation selon l'une des revendications précédentes, **caractérisée en ce qu'**elle comprend :
- 5 à 40% en poids du poids total de la composition de l'extrait aqueux de pollen ;
- 20 à 80% en poids du poids total de la composition de l'extrait aqueux de graines de *Cucurbita* ; et
- 0,1 à 10% en poids du poids total de la composition de vitamine E ou ses esters.

4. Composition pour son utilisation selon l'une des revendications précédentes, **caractérisée en ce qu'**elle comprend 0,1% à 10% d'extrait aqueux de pistil de *Zea mays* L. en poids du poids total de l'extrait.

5. Composition pour son utilisation selon l'une des revendications précédentes, **caractérisée en ce qu'**elle comprend en outre un actif choisi parmi du manganèse, des extraits de *Betula alba, Cerasus avium, Equisetum arvense, Phaseolus vulgaris, Achillea millefolium, Agropyron repens, Galium* verum, *Lavandula officinalis, Mentha piperita, Urticaria dioica.*

6. Composition pour son utilisation selon l'une des revendications précédentes, **caractérisée en ce qu'**elle est sous une forme adaptée pour une administration par voie orale.

7. Composition pour son utilisation selon la revendication 6, **caractérisée en ce qu'**elle est formulée sous forme de comprimé, gélule, capsule, gel mou, semi-solide, solide, liquide ou poudre.

8. Composition pour son utilisation selon la revendication 1, dans le traitement de l'incontinence urinaire à l'effort, de l'incontinence urinaire par urgenturie (ou impériosité) et de l'incontinence urinaire mixte.

9. Composition pour son utilisation selon la revendication 8, dans la réduction de la fréquence mictionnelle nocturne induisant un réveil.

## Patentansprüche

1. Zusammensetzung, umfassend:
- einen Pollenextrakt, der aus einem wässrigen Pollenextrakt besteht, der aus einer Mischung erhalten wird von:
- zu 45 Gew.-% bis 90 Gew.-% den wässrigen Pollenextrakt aus Secale cereale L. bezogen auf das Gesamtgewicht des Extrakts;
- zu 1 Gew.-% bis 35 Gew.-% den wässrigen Pollenextrakt aus Zea *mays L.* bezogen auf das Gesamtgewicht des Extrakts;
- zu 0,01 Gew.-% bis 5 Gew.-% den wässrigen Pollenextrakt aus *Pinus sylvestris* L. bezogen auf das Gesamtgewicht des Extrakts; und
- zu 3 Gew.-% bis 30 Gew.-% den wässrigen Pollenextrakt aus *Dactylis glomerata L.* bezogen auf das Gesamtgewicht des Extrakts,
- einen wässrigen Extrakt aus einem Stempel von *Zea mays L.,*
- einen wässrigen Extrakt aus Samen von (einer) Pflanze(n), die zu der Gattung *Cucurbita* gehört/gehören, der Fettsäuren mit einem Gehalt von weniger als 10 Gew.- % bezogen auf das Gesamtgewicht des Extrakts enthält, und
- Vitamin E oder seine Ester,
zur Verwendung bei der Behandlung von Harninkontinenz bei Frauen.

2. Zusammensetzung zur Verwendung nach Anspruch 1,
**dadurch gekennzeichnet, dass** der wässrige Extrakt aus Samen von *Cucurbita* Fettsäuren mit einem Gehalt zwischen 5 und 8 Gew.- % bezogen auf das Gesamtgewicht des Extrakts enthält.

3. Zusammensetzung zur Verwendung nach einem der vorstehenden Ansprüche, **dadurch gekennzeichnet, dass** sie umfasst:
- zu 5 bis 40 Gew.- % bezogen auf das Gesamtgewicht der Zusammensetzung den wässrigen Pollenextrakt;
- zu 20 bis 80 Gew.- % bezogen auf das Gesamtgewicht der Zusammensetzung den wässrigen Extrakt aus Samen von *Cucurbita*; und
- zu 0,1 bis 10 Gew.- % bezogen auf das Gesamtgewicht der Zusammensetzung von Vitamin E oder seinen Estern.

4. Zusammensetzung zur Verwendung nach einem der vorstehenden Ansprüche, **dadurch gekennzeichnet, dass** sie zu 0,1 Gew.-% bis 10 Gew.-% den wässrigen Extrakt aus dem Stempel von Zea *mays* L. bezogen auf das Gesamtgewicht des Extrakts umfasst.

5. Zusammensetzung zur Verwendung nach einem der vorstehenden Ansprüche, **dadurch gekennzeichnet, dass** sie ferner einen Wirkstoff umfasst, der aus Mangan, Extrakten aus *Betula alba, Cerasus avium, Equisetum* arvense, *Phaseolus vulgaris, Achillea millefolium, Agropyron repens,* Galium *verum, Lavandula officinalis, Mentha piperita, Urticaria* dioica ausgewählt ist.

6. Zusammensetzung zur Verwendung nach einem der vorstehenden Ansprüche, **dadurch gekennzeichnet, dass** sie in einer Form vorliegt, die für eine Verabreichung auf oralem Weg angepasst ist.

7. Zusammensetzung zur Verwendung nach Anspruch 6,
**dadurch gekennzeichnet, dass** sie in Form einer Tablette, einer Hartkapsel, einer Kapsel, eines weichen Gels, halbfest, fest, einer Flüssigkeit oder eines Pulvers ist.

8. Zusammensetzung zur Verwendung nach Anspruch 1 bei der Behandlung von Harnstressinkontinenz, Harninkontinenz durch Harndrang (oder Drang) und gemischter Harninkontinenz.

9. Zusammensetzung zur Verwendung nach Anspruch 8, wobei die Verringerung der Frequenz eines nächtlichen Wasserlassens ein Aufwecken bewirkt.

## Claims

1. Composition comprising :
- a pollen extract consisting of an aqueous pollen extract obtained from a mixture of :
- 45% to 90% aqueous extract of pollen of Secale cereale L. by weight of the total weight of the extract;
- 1% to 35% aqueous extract of pollen of Zea *mays* L. by weight of the total weight of the extract;
- 0.01% to 5% aqueous extract of pollen of *Pinus sylvestris L.* by weight of the total weight of the extract;
- 3% to 30% aqueous extract of pollen of *Dactylis glomerata L.* by weight of the total weight of the extract,
- an aqueous extract of the pistil of *Zea mays L.,*
- an aqueous extract of plant seeds belonging to the genus *Cucurbita* containing fatty acids at a content of less than 10% by weight of the total weight of the extract, and
- vitamin E or its esters,
for use in the treatment of urinary incontinence in women.

2. Composition for use according to claim 1, **characterised in that** the aqueous extract of *Cucurbita* seeds contains fatty acids at a content of between 5 and 8% by weight of the total weight of the extract.

3. Composition for use according to any one of the preceding claims, **characterised in that** it comprises :
- 5 to 40% of the aqueous pollen extract by weight of the total weight of the composition;
- 20 to 80% of the aqueous extract of plant seeds belonging to the genus Cucurbita by weight of the total weight of the composition; and
- 0,1 to 10% of vitamin E or its esters by weight of the total weight of the composition.

4. Composition for use according to any one of the preceding claims, **characterised in that** it comprises 0.1% to 10% of aqueous extract of the pistil of *Zea mays L.* by weight of the total weight of the extract.

5. Composition for use according to any one of the preceding claims, **characterised in that** it further comprises an active agent chosen from manganese, extracts of *Betula alba, Cerasus avium, Equisetum arvense, Phaseolus vulgaris, Achillea millefolium, Agropyron repens, Galium verum, Lavandula officinalis, Mentha piperita, Urticaria dioica.*

6. Composition for use according to any one of the preceding claims, **characterised in that** it is in a form suitable for oral administration.

7. Composition for use according to claim 6, **characterised in that** it is formulated in the form of a tablet, capsule, soft capsule, soft gel, semi-solid, solid, liquid or powder.

8. Composition for use according to claim 1 for the treatment of stress urinary incontinence, urgent urinary incontinence (or urge) and mixed urinary incontinence.

9. Composition for use according to claim 8, for reducing the nocturnal micturition frequency inducing an awakening.
